# EUROPEAN PATENT APPLICATION

(11) **EP 3 603 518 A1**
(43) Date of publication of application: **05.02.2020**
(21) Application number: 18775487.4
(22) Date of filing: 27.03.2018
(51) Int. Cl.: A61B 6/00, G06T 1/00, G06T 7/00

(54) **DIAGNOSTIC IMAGE PROCESSING APPARATUS, ASSESSMENT ASSISTANCE METHOD, AND PROGRAM**

(30) Priority: 27.03.2017 JP 2017061433
(71) Applicant: The University of Tokyo, Bunkyo-ku Tokyo 113-8654 (JP)
(72) Inventor: OKA Hiroyuki, Tokyo 113-8654 (JP); MATSUDAIRA Kou, Tokyo 113-8654 (JP); TANAKA Sakae, Tokyo 113-8654 (JP)
(74) Representative: SR Huebner - Munich Patentanwaltspartnerschaft mbB
(86) International application number: PCT/JP2018/012515
(87) International publication number: WO 2018/181363

(57) **Abstract**

This diagnostic image processing apparatus receives an input of an X-ray image of a hand or a foot, detects, in the X-ray image, the imaging range of the left hand, the right hand, the left foot, or the right foot, and extracts, at least for each joint located in the detected imaging range and used for assessment by using an assessment method selected from either van der Heijde method or Genant method, portions of relatively high luminance from the opposing ends of a pair of bones located across each of the joints. Then, the diagnostic image processing apparatus generates and outputs information about a distance and an area between the extracted high luminance parts as diagnosis information for each joint used for the assessment carried out by the selected assessment method.

## Description

### Technical Field

The present disclosure relates to a diagnostic image processing apparatus, an assessment assistance method, and a program.

### Background Art

In diagnosis of rheumatoid arthritis, the Sharp method in which the joint space is evaluated with a five-point scale, using an X-ray image, is widely known (Non-Patent Document 1). When this method is used, each space is evaluated with respect to joints determined as joints to be assessed, by van der Heijde (VDH) method or Genant method.

### Prior Arts

### Non-Patent Document

Non-Patent Document 1: Sharp JT, et. al., "The progression of erosion and joint space narrowing scores in rheumatoid arthritis during the first twenty-five years of disease.", Arthritis Rheum. 1991 Jun; 34(6): 660-8.

### Summary

### Problems to be Solved by the Invention

However, in the above prior methods, the size of the joint space is visually assessed by a medical doctor, and assessment results may be varied depending on the medical doctor. Therefore, a reference image is prepared, but in some diseases such as rheumatism, a bone itself is deformed, and a comparison with the reference image becomes difficult.

The present disclosure has been made with reference with the above, and one of the objectives of the present disclosure is to provide a diagnostic image processing apparatus, an assessment assistance method, and a program capable of assessing the size of a predetermined joint space in a quantitative way.

### Solving Means

In order to solve the above problems, the present disclosure provides a diagnostic image processing apparatus comprising: a receiving device which receives an input of an X-ray image of a hand or a foot, a high luminance portion extraction device which detects a captured image range of the left hand, the right hand, the left foot, or the right foot from the received X-ray image, and with respect to at least each joint to be used by an assessment method selected from either van der Heijde (VDH) or Genant from among the joints of the left hand, the right hand, the left foot, or the right foot located in the detected captured image range, extracts portions having relatively high luminance from opposing ends of a pair of bones having the joint therebetween, a diagnostic information generation device which generates information regarding a distance and an area between the extracted high luminance portions as diagnostic information, with respect to each joint to be used for assessment by the selected assessment method, and an output device which outputs the generated diagnostic information.

### Effect of the Invention

Thereby, the size of a predetermined joint space can be assessed in a quantitative way.

### Brief Description of Drawings

FIG. 1 is a structural block diagram showing an example of a diagnostic image processing apparatus according to an embodiment of the present disclosure.
FIG. 2 is a functional block diagram showing an example of a diagnostic image processing apparatus according to an embodiment of the present disclosure.
FIG. 3 is an explanatory view showing an example of preprocessing by a diagnostic image processing apparatus according to an embodiment of the present disclosure.
FIG. 4 is an explanatory view showing an example of processing by a diagnostic image processing apparatus according to an embodiment of the present disclosure.
FIG. 5 is an explanatory view showing an example of diagnostic information generation process by a diagnostic image processing apparatus according to an embodiment of the present disclosure.
FIG. 6 is an explanatory view showing an output example of diagnostic information by a diagnostic image processing apparatus according to an embodiment of the present disclosure.
FIG. 7 is a flowchart showing a process flow by a diagnostic image processing apparatus according to an embodiment of the present disclosure.
FIG. 8 is an explanatory view showing an example of information used in a diagnostic image processing apparatus according to an embodiment of the present disclosure.

### Embodiment

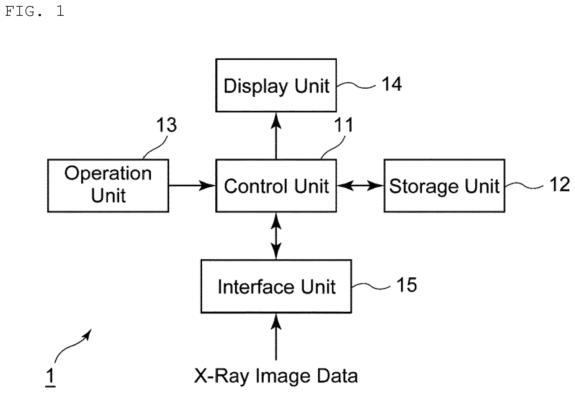

An embodiment of the present disclosure will be explained with reference to the drawings. As exemplified in FIG. 1a diagnostic image processing apparatus according to an embodiment of the present disclosure 1 comprises a control unit 11, a storage unit 12, an operation unit 13, a display unit 14, and an interface unit 15.

The control unit 11 is a program-controlled device such as a CPU, and operates in accordance with a program stored in the storage unit 12. In the present embodiment, the control unit 11 receives, through the interface unit 15, image data of a captured X-ray image of at least one of the left hand, the right hand, the left foot, and the right foot. From the received X-ray image, the control unit 11 detects a captured image range of any of the left hand, the right hand, the left foot, or the right foot. Then, with respect to at least each joint to be used for an assessment by a method selected from either the van der Heijde (VDH) method or the Genant method from among the joints of the left hand, the right hand, the left foot, or the right foot taken in the detected image capture range, portions having relatively high luminance are extracted from the opposing ends of a pair of bones with the joint therebetween. With respect to each joint to be used for the assessment by the selected assessment method, the control unit 11 generates distance and area information between the extracted high luminance portions, as diagnostic information, and outputs the generated diagnostic information. The processes by the control unit 11 will be explained in detail below.

The storage unit 12 is a memory device, a disk device, etc., which stores a program to be executed by the control unit 11. The program may be provided by being stored in a computer-readable non-transitory storage medium, and installed in the storage unit 12. Further, the storage unit 12 may operate as a work memory of the control unit 11.

The operation unit 13 is a mouse, a keyboard, etc., which receives an instruction operation from a user, and outputs the content of the instruction operation to the control unit 11. The display unit 14 is a display, etc., which displays and outputs information in accordance with the instruction input from the control unit 11.

The interface unit 15 includes a serial interface such as USB (Universal Serial Bus), etc., and a network interface, which receives various data from a portable medium such as a memory card, etc., an external PC, and the like, and outputs the received data to the control unit 11. According to an example of the present embodiment, the interface unit 15 receives, from an external apparatus, an input of image data of an X-ray image to be processed, and outputs the received data to the control unit 11.

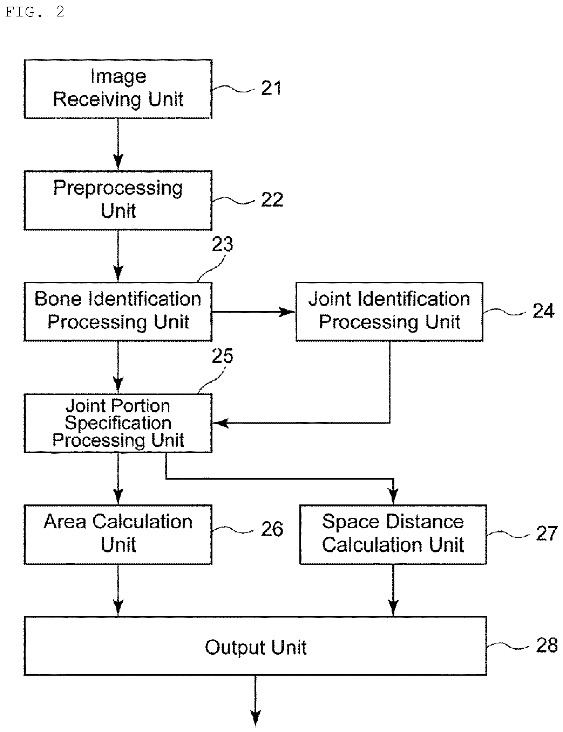

Then, operations of the control unit 11 will be explained. According to an example of the present embodiment, as exemplified in FIG. 2, the control unit 11 functionally comprises an image receiving unit 21, a preprocessing unit 22, a bone identification processing unit 23, a joint identification processing unit 24, a joint portion specification processing unit 25, an area calculation unit 26, a space distance calculation unit 27, and an output unit 28.

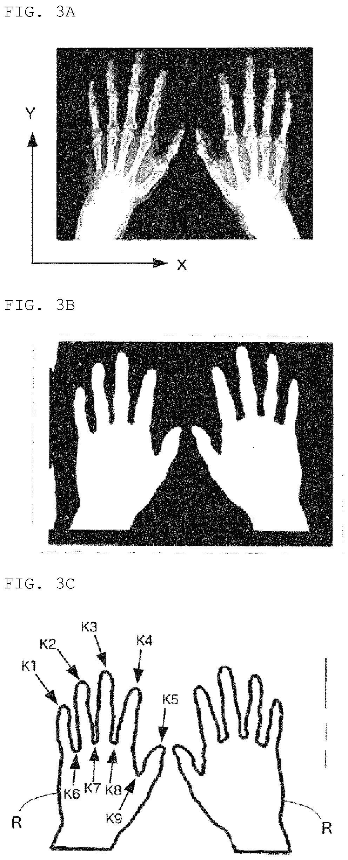

The image receiving unit 21 receives an input of image data of an X-ray image to be processed. Here, the image data to be input is, for example, as shown in FIG. 3A, data of an X-ray image of both hands captured while the hands are arranged to be juxtaposed in the transverse direction (X-axis direction), with the palms of the hands facing downward. In FIG. 3, the transverse axis is X-axis, and the vertical axis is Y-axis.

The preprocessing unit 22 applies contrast adjustment processes, such as a process of reducing noise by a median filter, a process of making outlines clear by Robert filter, and the like, to the image data to be processed. The image data to be processed, after being subjected to the contrast adjustment processes, is binarized at a predetermined luminance threshold (for example, 50%) (FIG. 3B)

With respect to the binarized image data to be processed, the preprocessing unit 22 further repeats dilation of significant pixels (here, pixels with high luminance) (when a non-significant pixel P is adjacent to a significant pixel, the pixel P is set to a significant pixel), for N times (for example, N=3). After the dilation process of the significant pixels, the preprocessing unit 22 repeats an erosion process (when a significant pixel P is adjacent to a non-significant pixel, the pixel P is set to a non-significant pixel) for N times, to thereby perform a closing process. Therefore, when a significant pixel region of the binarized image data to be processed partially includes a non-significant part, the non-significant part is treated as significant pixels, and the entirety of the left hand and the entirety of the right hand are defined.

Then, the preprocessing unit 22 executes a process of extracting outlines to extract outlines R each surrounding the entirety of the left hand and the entirety of the right hand, respectively (FIG. 3C). Note that, for the purpose of explanation, only the outlines detected from FIG. 3B are shown in FIG. 3C. Further, the preprocessing unit 22 performs labeling of each region surrounded by the outline R, and identifies the region corresponding to either of the hands as an image-captured region of the hand to be processed. Specifically, in the following example, the hand with its little finger lying on the negative direction of X-axis (the left in the Figure) is to be subjected to the following processes. Accordingly, in the processing of an image of the right hand, the image data to be processed is reflected over the Y-axis (right-left) before the image is subjected to the following processes.

Namely, the preprocessing unit 22 treats a region r1 surrounded by the outline R and located at the negative side in the X-axis direction, as a region to be processed. Here, when the image is reflected, the hand to be processed is the right hand, whereas when the image is not reflected, the hand to be processed is the left hand. The preprocessing unit 22 outputs information representing the region of the captured-image of the hand to be processed (information specifying the outline R which surrounds the region r1), and the image data to be processed after being subjected to the contrast adjustment process.

The bone identification processing unit 23 receives the input of information representing the region of the captured-image of the hand to be processed, and the image data to be processed after being subjected to the contrast adjustment process, which have been output by the preprocessing unit 22. The bone identification processing unit 23 identifies, from among the bones captured in the image of the received image data to be processed after being subjected to the contrast adjustment process, an image-captured range of each bone within the image-captured region of the hand to be processed, and then, on the basis of the identification results and the position information of the identified range, performs a labeling process for specifying the bones represented by the image-captured bones in each range.

Specifically, the bone identification processing unit 23 identifying bones as follows. The bone identification processing unit 23 estimates the length in the longitudinal direction of each bone in the finger. For this estimation, the bone identification processing unit 23, first, refers to the information representing the image-captured region of the hand to be processed, i.e., the information specifying the outline R surrounding the region, and detects inflection points of the outline (tops of upward convexes in the Y-axis direction (from K1 to K5 in the order from the negative side of the X-axis in FIG. 3C) or tops of downward convexes (from K6 to K9 in the order from the negative side of the X-axis in FIG. 3C). For the detection of inflection points, a widely known method can be applied. Here, the tops of the upward convexes (K1 to K5) correspond to fingertips, and the tops of the downward convexes (K6 to K9) correspond to crotches between the fingers.

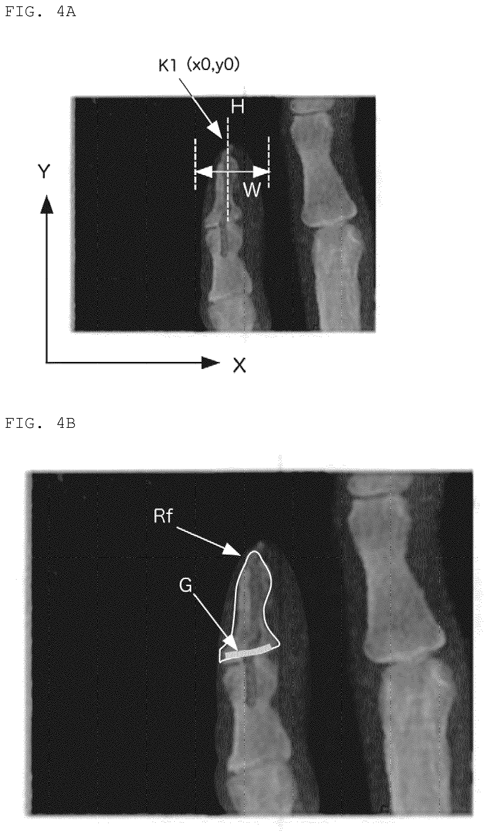

Then, the bone identification processing unit 23 extracts an outline of each bone of the finger. For example, the bone identification processing unit 23 first obtains the center line of each bone (FIG. 4A). FIG. 4A shows an enlarged view of the tip of the little finger. For example, the center line is obtained as follows. Namely, the bone identification processing unit 23 sets the initial position at a coordinate (x₀, y₀) located on the image data to be processed of the fingertip corresponding to each finger (any one of K1 to K5, and K1 in case of FIG. 4A). Then, the bone identification processing unit 23 scans in the direction from this initial position toward the proximal hand (in the direction that the Y-axis value decreases) by one pixel at each time, and obtains X coordinate values (x_{jL}, x_{jR}) of the right and left points located on each line segment yⱼ=yⱼ₋₁-1 (j=1, 2, ...) which is in parallel with the X-axis and on the outline of the bone within a predetermined width W with X=xⱼ₋₁ at the center (the point being selected so that the absolute value of the difference in luminance from the adjacent pixel is a predetermined threshold value or larger).

The bone identification processing unit 23 obtains an average value (median point) of the above-obtained X coordinate value as X coordinate value x_{jw}= (x_{jL}+x_{jR}) /2, and obtains xⱼ in a way so that:
when xⱼ₋₁<x_{jw}, xⱼ=xⱼ₋₁+1,
when xⱼ₋₁=x_{jw}, xⱼ=xⱼ₋₁, and
when xⱼ₋₁>x_{jw}, xⱼ=xⱼ₋₁-1.

The bone identification processing unit 23 repeatedly executes the processes until luminance of a pixel located at (xⱼ₋₁, yⱼ) is determined as exceeding a predetermined luminance threshold value (having a higher luminance), while incrementing j by 1. Thereby, the bone identification processing unit 23 obtains the center line H of the distal phalanx of each finger.

The bone identification processing unit 23 extracts a pixel block in a rectangle defined by a width W and a height |y₀-y_{J}| with the coordinate (x₀, y₀) of the initial position at the center of the upper side (with the proviso that j refers to the first value of j when the luminance of the pixel located at (xⱼ₋₁, yⱼ) exceeds a predetermined luminance threshold value, and |a| refers to an absolute value of a). The bone identification processing unit 23 performs affine transformation so that the center line within the pixel block becomes in parallel with the Y-axis, and performs a closing process regarding the image data in the pixel block after the affine transformation. Further, the bone identification processing unit 23 extracts an outline Rf on the basis of the image data after the closing process by a method such as Sobel filter, etc. With respect to the outline on the upper and lower sides in the Y-axis direction (in case of the distal phalanx, the outline on the lower side in the Y-axis direction), a portion having luminance exceeding a predetermined luminance threshold value (high luminance portion G) is extracted as a part of the outline (FIG. 4B). Thereby, the bone identification processing unit 23 extracts the outline of the distal phalanx of each finger.

The bone identification processing unit 23 extracts the portions having luminance exceeding a predetermined luminance threshold value as the outlines on the upper and lower sides in the Y-axis direction, because a bone has relatively hard portions formed at positions sandwiching the joint, and captured images of the hard portions are portions having a relatively high luminance.

Further, the bone identification processing unit 23 continues processes that the center of the lower side of the outline of the bone detected for each finger (or a point located on the lower side of the pixel block and having an X-axis value same as the X-axis value of the center line detected in the pixel block) is set as an initial position candidate; a pixel which is located at a position moved downward from this initial position candidate and in parallel with the Y-axis, and which has luminance exceeding the predetermined luminance threshold value while a pixel right above in the Y-axis direction has luminance lower than the predetermined luminance value, is treated as the center on the upper side of the bone located at the proximal of the distal phalanx, and the position of this pixel is set as the initial position; a center line is recognized and a rectangle surrounding the center line is set; an image block in the rectangle is subjected to affine transformation so that the center line becomes in parallel with the Y-axis; and the closing process is performed to extract an outline.

Accordingly, with respect to the thumb, outlines of distal phalanx → proximal phalanx → metacarpal are successively extracted, and image portions of respective bones surrounded by the extracted outlines are labeled with information specifying the corresponding bones. Further, with respect to each of the index finger, the middle finger, the ring finger, and the little finger, outlines of distal phalanx → middle phalanx → proximal phalanx → metacarpal are successively extracted, and image portions of respective bones surrounded by the extracted outlines are labeled with information specifying the corresponding bones.

The joint identification processing unit 24 labels a space region between the image portion labeled by the bone identification processing unit 23 and the image portion labeled as a bone adjacent thereto, as a region where an image of a corresponding joint portion is captured.

According to the present embodiment, by the above-mentioned method, the portion having relatively high luminance (high luminance portion) in the opposing ends of a pair of bones having the joint therebetween, is extracted as an outline of the bone adjacent to the joint. Therefore, the joint identification processing unit 24 identifies the region sandwiched from above and below by the relatively high luminance portions of the mutually adjacent bones, the captured images of them being in the image data to be processed, (a circumscribed rectangle region including a relatively high luminance portion in a lower part of the distal side bone, and a relatively high luminance portion in a upper part of the proximal side bone, of the mutually adjacent pair of bones) as a joint portion. Further, on the basis of labeling information of the bones located at the upper and lower sides of the identified joint portion, the joint identification processing unit 24 identifies a joint corresponding to the region in the image data to be processed including a captured image of the identified joint portion, and records the name of the corresponding joint in association with each region.

Namely, in this example of the present embodiment, a high luminance portion extraction device is realized by the bone identification processing unit 23 and the joint identification processing unit 24.

The joint portion specification processing unit 25 receives an input of selecting either VDH of Genant as a diagnostic assessment method from a user. With respect to each joint to be used for the selected assessment method among the joints identified by the joint identification processing unit 24, the joint portion specification processing unit 25 extracts an image portion in the region in the corresponding image data to be processed, and outputs the extracted image portion to the area calculation unit 26 and the space distance calculation unit 27.

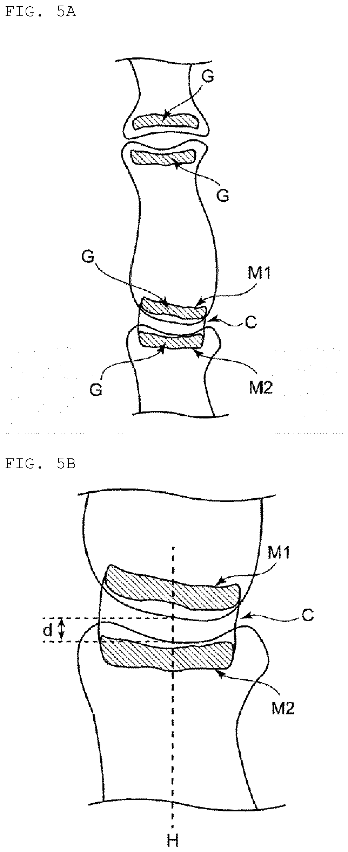

On the basis of each image portion output from the joint portion specification processing unit 25, the area calculation unit 26 specifies pixels of an image portion having relatively high luminance (high luminance portion), and obtains an area (number of pixels) of a portion between the specified pixels. For example, the area calculation unit 26 performs a closing process for the image portion output from the joint portion specification processing unit 25, and extracts an outline of relatively high luminance pixels in the image portion subjected to the closing process. As mentioned above, images of portions of a pair of mutually adjacent bones having a joint therebetween are captured to have relatively high luminance. Thus, as exemplified in FIG. 5A, normally, two outlines M1, M2 are extracted. The area calculation unit 26 obtains a convex hull C of pixels included in the two extracted outlines M1, M2. The area calculation unit 26 outputs the number of pixels obtained by subtracting pixels having luminance higher than a predetermined luminance threshold value (high luminance portion), as an image portion having a relatively high luminance, from this convex hull C, and outputs the obtained number of pixels as area information.

The area calculation unit 26 performs these processes of obtaining the area information and outputting the obtained information, with respect to each image portion output by the joint portion specification processing unit 25. Thereby, the diagnostic image processing apparatus 1 according to the present embodiment obtains information of the area between the high luminance portions with respect to each joint to be used for the assessment by the selected assessment method, i.e., either VDH or Genant.

The space distance calculation unit 27 specifies pixels of captured image portions with relatively high luminance (high luminance portions) on the bases of the image portions output from the joint portion specification processing unit 25, and obtains a distance between the specified pixels. Specifically, similar to the area calculation unit 26, the space distance calculation unit 27 performs a closing process for the image portion output from the joint portion specification processing unit 25, and extracts a pair of outlines M1, M2 of the relatively high luminance pixels in the image portion subjected to the closing process. Then, as exemplified in FIG. 5B, with respect to a proximal side bone among the bones the captured images of which are included in the image portion, the space distance calculation unit 27 counts the number of pixels which are located on the extension of the center line H detected by the bone identification processing unit and are located between the pair of outlines M1, M2, and outputs the value obtained by counting as a distance d between the high luminance portions.

The space distance calculation unit 27 these processes with respect to each image portion output by the joint portion specification processing unit 25. Thereby, the diagnostic image processing apparatus 1 according to the present embodiment obtains information of the distance between the high luminance portions with respect to each joint to be used for the assessment by the selected assessment method, i.e., either VDH or Genant.

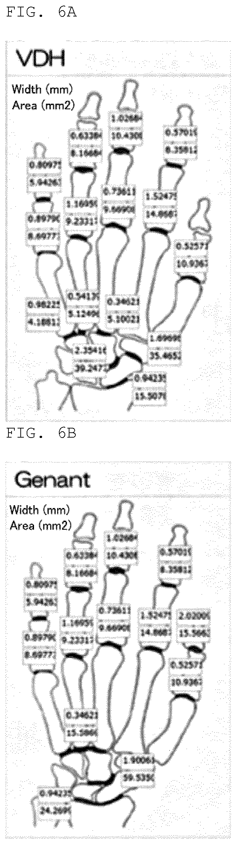

The output unit 28 outputs the area information and the distance information output by the area calculation unit 26 and the space distance calculation unit 27, with respect to each joint to be used for the assessment by the selected assessment method, i.e., either VDH or Genant, in association with information specifying the corresponding joint. Specifically, the output unit 28 functions so that the display unit 14 displays information as exemplified in FIG. 6. FIG. 6A shows an example when VDH is selected, and FIG. 6B shows an example when Genant is selected. Further, the output unit 28 may convert the area information and the distance information obtained from the area calculation unit 26 and the space distance calculation unit 27 to square millimeter unit and millimeter unit, before the output. Such output can be easily calculated by using the actual length (millimeter) information (received by separately performed input) of a pixel.

### [Operation]

The diagnostic image processing apparatus 1 according to the present embodiment is constituted as above, and operates as below. Namely, the diagnostic image processing apparatus 1 according to the present embodiment receives image data of an X-ray image having a captured image of at least one of the left hand, the right hand, the left foot, or the right foot. Also, the diagnostic image processing apparatus 1 receives instructions from a user as to which assessment method is to be used, VDH or Genant. Further, according to the following example of the present embodiment, information regarding the size of one pixel (actual length (millimeter) of the height or the width) of the X-ray image in the image data, is previously set.

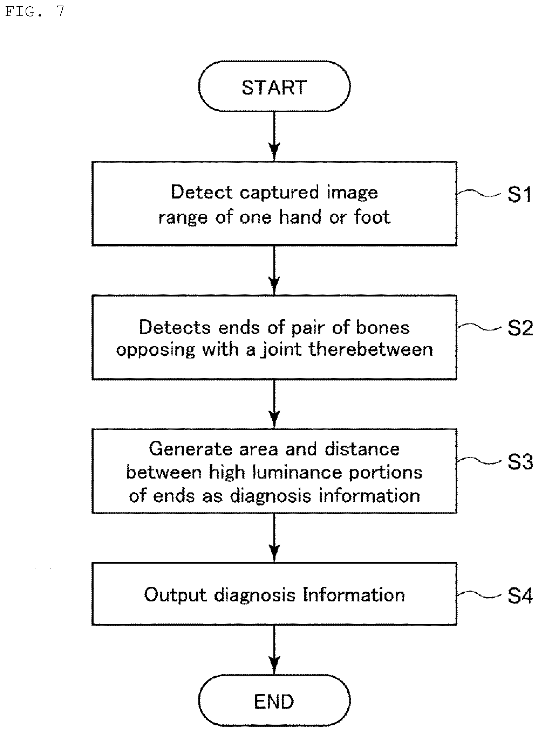

As exemplified in FIG. 7, the diagnostic image processing apparatus 1 detects a captured image range of the left hand, the right hand, the left foot, of the right foot, using the received X-ray image as an image data to be processed (S1). Here, as exemplified in FIG. 3A, the input image data is an X-ray image captured while both hands are juxtaposed in the X-axis direction with the palms facing downward, and the diagnostic image processing apparatus 1 determines, for example, the hand located on the negative side in the X-axis, as the hand to be processed.

The diagnostic image processing apparatus 1 detects a joint-side end of each of a pair of bones opposing with the joint therebetween, with respect to each join of the fingers of the hand to be processed (S2). Here, the end is detected by extracting a portion having relatively high luminance (high luminance portion).

Further, the diagnostic image processing apparatus 1 specifies a bone including the extracted high luminance portion. The identification of the bone (identification of the bone, and the finger that the bone belongs to) is performed on the basis of the detected position of each bone. Namely, the diagnostic image processing apparatus 1 detects inflection points (tops of upward convexes in the Y-axis direction (K1 to K5 in FIG. 3C) or tops of downward convexes (K6 to K9 in FIG. 3C) in the outline of the hand to be processed, determines the coordinate (x₀, y₀) of the top of the finger (each of K1 to K5) corresponding to each finger as an initial position. Then, an outline including the initial position is obtained, and the obtained outline is determined as an outline of the most distal side bone (distal phalanx) of each finger.

In the outline of the distal phalanx, the center coordinate in the X-axis direction, on the side closer to the proximal side bone, is determined. A line segment extending downward from the center coordinate and in parallel with the Y-axis, is determined, and the outline of the next bone is detected on the line segment. Further, the outline of the bone located next proximal side of the distal phalanx is detected for each finger. The diagnostic image processing apparatus 1 repeats the processes to detect the outline of each bone of each finger.

Accordingly, with respect to the thumb, outlines of the distal phalanx → proximal phalanx → metacarpal are sequentially extracted, and the image portion of each bone surrounded by the extracted outline is labeled with information specifying the corresponding bone. Further, with respect to each of the index finger, the middle finger, the ring finger, and the little finger, outlines of the distal phalanx → middle phalanx → proximal phalanx → metacarpal are sequentially extracted, and the image portion of each bone surrounded by the extracted outline is labeled with information specifying the corresponding bone

Next, the diagnostic image processing apparatus 1 detects a high luminance pixel block located at the joint-side end of the image portion of each bone that has been labeled. Then, the diagnostic image processing apparatus 1 determines information specifying the joint adjacent to the detected high luminance portion, on the basis of the information specifying the bone including the high luminance portion. For example, the joint located between the high luminance portions respectively included in the distal phalanx and the proximal phalanx of the thumb, is determined as the IP joint. Further, the joint located between the high luminance portions respectively included in the middle phalanx and the proximal phalanx of the index finger is determined as the PIP joint. Then, the diagnostic image processing apparatus 1 stores the information for specifying the pixel group of the extracted high luminance portion in association with the information for specifying the joint adjacent to the high luminance portion.

Further, with respect to each joint that is to be used for assessment by a selected assessment method, i.e., VDH or Genant, the diagnostic image processing apparatus 1 generates information of the distance and the area of the space between the high luminance portions located at the opposing ends of the pair of bones having the joint therebetween (S3). Then, the diagnostic image processing apparatus 1 converts the generated diagnostic information to a unit of the actual length (square millimeter and millimeter), and outputs the converted information as diagnostic information of the left hand (S4), as exemplified in FIG. 6.

Here, the area and the distance of the joint portion are obtained as diagnostic information, but other information may be used in place thereof, or in addition thereto.

Further, when there are captured images of other hand or foot, on the outside of the captured image range detected in Step S1, the diagnostic image processing apparatus 1 may detect the captured image range other hand or foot and perform processes by repeating from Step S1. Specifically, according to the present embodiment, out of the captured images of juxtaposed hands or feet, the hand or foot located on the negative side of the X-axis is to be processed. When there is a captured image of the other hand or foot on the positive side of the X-axis, the image is reflected in the X-axis direction (axially symmetric with respect to the Y-axis), and the processes are repeated again from Step S1. In this case, the diagnostic information output in Step S4 is diagnostic information of the right hand.

The way of recognizing the high luminance portion is not limited to the way in the above example. For example, relationship between the image data to be processed in which the region of the high luminance portion has already been fixed and the relevant fixed region of the high luminance portion can be learned by machine learning using a multilayer neural network, and the region of the high luminance portion can be recognized by the trained machine learning multilayer neural network.

Also, an outline of a portion having luminance higher than a predetermined threshold value is extracted, and the relationship between the position of the extracted outline and the information specifying the joint (information indicating the first joint of thumb (IP), and the like) can be learned by machine learning using a multilayer neural network. The region of the high luminance portion and the information specifying the joint can be obtained by the trained machine learning multilayer neural network.

### [Modified Example for Detecting Outline of Bone]

In the above-mentioned bone identification processing unit 23, when an outline of a bone is detected, probability of errors in the outline detection can be decreased, by taking the length of the finger into account. Specifically, the bone identification processing unit 23 according to the present example calculates the distance between the detected fingertip and the crotch of the finger, and obtains information regarding the length of the longest finger. Here, the bone identification processing unit 23 generates a virtual line segments L1, L2, L3 connecting between adjacent tops of downward convexes, i.e., between K6 and K7, K7 and K8, and K8 and K9, respectively. Next, out of the tops K1 to K5, the shortest distance Z1 from the top K2 to the line segment L1, the shortest distance Z2 from the top K3 to the line segment L2, the shortest distance Z3 from the top K4 to the line segment L3 are obtained. The longest of the obtained distances (normally, the middle finger is the longest, and thus, the shortest distance Z2 from the top K3 to the line segment L2 is the longest) is determined as information of the length of the longest finger.

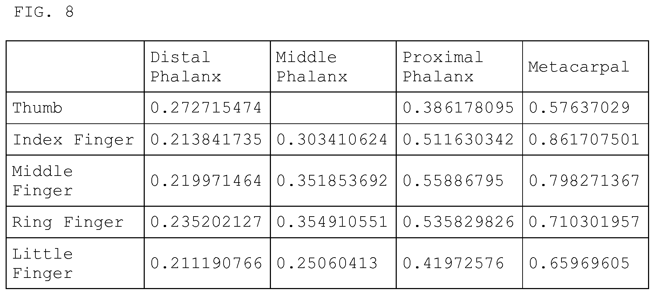

The bone identification processing unit 23 uses this information of the length of the longest finger to estimate the length, in the longitudinal direction, of each bone of the fingers. Namely, the bone identification processing unit 23 refers to information regarding the ratio of length in the longitudinal direction of each bone, relative to the length of the longest finger, in terms on an ordinary human, the information being previously recorded in the storage unit 12. As exemplified in FIG. 8, the information includes, for example, the ratios in length of the distal phalanx, the proximal phalanx, and the metacarpal of the thumb, relative to the length of the longest finger; and the ratios in length of the distal phalanx, the middle phalanx, the proximal phalanx, and the metacarpal of each of the index finger, the middle finger, the ring finger, and the little finger, relative to the length of the longest finger.

When extracting an outline of a bone having its upper and lower direction in the Y-axis direction, the bone identification processing unit 23 can obtain the length in the upper-lower direction. If the obtained length is not within a predetermined ratio range (a range including "1.0 (identical)", such as 0.8 to 1.2), relative to the length in the longitudinal direction of the estimated corresponding bone, the bone identification processing unit 23 can perform a process for adjusting contrast, such as averaging the contrast, etc., before repeating the process of extracting the outline of the bone again.

### [Correction of Outline]

The outline generated by the bone identification processing unit 23 for each bone can be corrected by a user. By way of an example, the diagnostic image processing apparatus 1 according to the present embodiment obtains an outline of a bone, and thereafter, performs fitting of the outline with a spline curve (for example, three-dimensional B spline curve). The method of fitting is widely known, and thus, the detailed explanation therefor is omitted here.

Then, the diagnostic image processing apparatus 1 draws the outline of each bone subjected to the fitting with a spline curve, on a corresponding position of the image data to be processed so as to overlap thereon, displays the outline on the display unit 14, receives an input of operations to move the positions of the control points of the spline curve from a user through the operation unit 13, and updates the content of the drawing by generating a spline curve on the basis of the positions of the control points after being moved. Thereby, the user can visually recognize the actual X-ray image data, while manually correcting the outline of the corresponding bone.

### [Recording]

Further, the diagnostic image processing apparatus 1 according to the present embodiment can receive an input of information specifying a person to be diagnosed (information for specifying a person whose image is captured in X-ray image data, such as name, etc.), and may record the generated diagnostic information in association with the input information for specifying a person to be diagnosed and time and date when the X-ray image data is captured (the input thereof being received separately), in a database (not shown).

In the present example, the diagnostic image processing apparatus 1 can generate and display statistical information or graph information showing the transition of the diagnostic information on the basis of the X-ray image data captured for the same person to be diagnosed at mutually differ plurality of time points (image captured date and time), and results of extrapolation calculation (predicted diagnostic information at a time point in the future), and the like. Such information can help doctors, etc., to understand not only the status of bone deformation, but also the status proceeding with time, i.e., status of progress or improvement of the deformation. Thus, assessment regarding the progress or improvement of bone deformation can be assisted.

Further, at this time, in addition to the diagnostic information, information regarding the outline of each bone (for example, information specifying a B spline curve fitted to the outline of the bone) can be recorded in the database, in association with the information for specifying a person to be diagnosed and time and date when the X-ray image data is captured.

Accordingly, with respect to the same person to be diagnosed, diagnostic information (outline information, etc.) obtained at mutually different plurality of time points (image captured date and time) can be compared, and thus, the change of the outline of each bone along the passage of time can be examined, and analysis of bone erosion can be performed. Namely, status of progress or improvement of deformation can be easily grasped, and assessment regarding the progress or improvement of bone deformation can be assisted.

### [Bone of Wrist]

In the above explanation, an example of generating diagnostic information such as area, etc., regarding the joint portion between the bones of the finger is described. However, in the assessment method such as VDH, Genant, etc., generating area, distance information regarding a space between the bones of the wrist is preferable.

In this case, because difference in the shape of the bone of the wrist is relatively large among different individuals, machine learning with a multilayer neural network can be performed to estimate the outline of each bone, and area information and distance information of the space can be generated on the basis of the estimated result and through appropriate adjustment of the outline position by a user.

### [Effect of Embodiment]

According to the present embodiment, on the basis of X-ray image data, near the outline of each bone of the hand or foot, relatively hard portions of bones which oppose with a joint therebetween are identified as high luminance portions, and area information and distance information of the part between the high luminance portions are calculated and displayed. Thereby, compared to the case where the status of the space is visually judged, numeral information regarding area and distance can be obtained, and thus, the size of the joint space can be quantitatively assessed.

### Explanation on Numerals

1 diagnostic image processing apparatus, 11 control unit, 12 storage unit, 13 operation unit, 14 display unit, 15 interface unit, 21 image receiving unit, 22 preprocessing unit, 23 bone identification processing unit, 24 joint identification processing unit, 25 joint portion specification processing unit, 26 area calculation unit, 27 space distance calculation unit, 28 output unit

## Claims

1. A diagnostic image processing apparatus comprising: a receiving device which receives an input of an X-ray image of a hand or a foot,
a high luminance portion extraction device which detects a captured image range of the left hand, the right hand, the left foot, or the right foot from the received X-ray image, and with respect to at least each joint to be used by an assessment method selected from either van der Heijde (VDH) or Genant from among the joints of the left hand, the right hand, the left foot, or the right foot located in the detected captured image range, extracts portions having relatively high luminance from opposing ends of a pair of bones having the joint therebetween,
a diagnostic information generation device which generates information regarding a distance and an area between the extracted high luminance portions as diagnostic information, with respect to each joint to be used for assessment by the selected assessment method, and
an output device which outputs the generated diagnostic information.

2. A diagnostic image processing apparatus according to claim 1 further comprising an outline setting device which detects a captured image range of the left hand, the right hand, the left foot, or the right foot from the received X-ray image, and sets an outline of each bone, an image of which is captured in the detected captured image range,
wherein
the high luminance portion extraction device specifies a bone including the extracted high luminance portion using the set outline, and outputs information for specifying a joint located adjacent to the high luminance portion together with information of the extracted high luminance portion, and
the diagnostic information generation device generates information regarding a distance and an area between the extracted high luminance portions adjacent to the specified joint, as diagnostic information, with respect to each joint to be used for assessment by the selected assessment method, and outputs the generated diagnostic information in association with information for specifying a joint regarding the generated diagnostic information.

3. A diagnostic image processing apparatus according to claim 2, wherein
the outline setting device estimates a length of the longest finger among the fingers the images of which are captured in the detected captured image range, estimates a length of each bone the image of which is captured in the detected captured image range using a predetermined ratio relative to the length of the estimated finger, and sets an outline of each bone using the estimated length.

4. A diagnostic image processing apparatus according to any one of claims 1 to 3, further comprising
a device which further receives an input of information for specifying a person to be diagnosed, the image of the person being captured in the received X-ray image, and an input of information of time and date when the X-ray image is captured, and which records the diagnostic information in association with the received information for specifying the person to be diagnosed and the information of image captured time and date,
so as to output a record of diagnostic information on the basis of X-ray images captured on mutually different time and date for each person to be diagnosed.

5. A method for assisting assessment of progress or improvement of bone deformation using a computer, comprising:
a step of inputting an X-ray image of a hand or a foot, information for specifying a person to be diagnosed whose image is captured in the X-ray image, and information of time and date when the X-ray image is captured; detecting a captured image range of the left hand, the right hand, the left foot, or the right foot from the X-ray image; and extracting, with respect to at least a joint to be used by an assessment method selected from either van der Heijde (VDH) or Genant from among the joints of the left hand, the right hand, the left foot, or the right foot located in the detected captured image range, portions having relatively high luminance from opposing ends of a pair of bones having the joint therebetween;
a step of generating information regarding a distance and an area between the extracted high luminance portions as diagnostic information;
a step of recording the generated diagnostic information in association with the received information for specifying the person to be diagnosed and the information of image captured time and date; and
a step of outputting a record of diagnostic information on the basis of X-ray images captured on mutually different time and date for each person to be diagnosed.

6. A program to function a computer as:
a receiving device which receives an input of an X-ray image of a hand or a foot,
a high luminance portion extraction device which detects a captured image range of the left hand, the right hand, the left foot, or the right foot from the received X-ray image, and with respect to at least each joint to be used by an assessment method selected from either van der Heijde (VDH) or Genant from among the joints of the left hand, the right hand, the left foot, or the right foot located in the detected captured image range, extracts portions having relatively high luminance from opposing ends of a pair of bones having the joint therebetween, a diagnostic information generation device which generates information regarding a distance and an area between the extracted high luminance portions as diagnostic information, and
an output device which outputs the generated diagnostic information.
